(19) Europäisches Patentamt
European Patent Office
Office européen des brevets

(11) Numéro de publication : **0 497 678 A1**

# (12) DEMANDE DE BREVET EUROPEEN

(21) Numéro de dépôt : **92400204.1**

(51) Int. Cl.[5] : **C07C 243/38,** C07D 271/10

(22) Date de dépôt : **27.01.92**

(30) Priorité : **30.01.91 FR 9101030**

(43) Date de publication de la demande :
**05.08.92 Bulletin 92/32**

(84) Etats contractants désignés :
**AT BE CH DE DK ES FR GB GR IT LI LU MC NL PT SE**

(71) Demandeur : **ELF ATOCHEM S.A.**
**4 & 8, Cours Michelet La Défense 10**
**F-92800 Puteaux (FR)**

(72) Inventeur : **Gillet, Jean-Phlippe**
**4, rue du Garel**
**F-69530 Brignais (FR)**

(54) **Diaryl-2,5, oxadiazoles-1,3,4, hydroxyesters, hydroxyacide et acétoxyacide, leur procédé de synthèse.**

(57) L'invention concerne les diaryl-2,5 oxadiazoles-1,3,4 à terminaisons hydroxyester, hydroxyacide et acétoxyacide de formule :

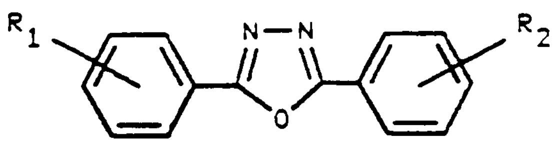

dans laquelle $R_1$ représente un radical hydroxyle ou acétyle et $R_2$ représente un radical carboxyle et où $R_1$ et $R_2$ occupent de préférence simultanément les positions 3 et 3′ ou 4 et 4′.
Les hydroxyesters sont préparés par cyclisation du dihydrazide diaromatique correspondant et les hydroxyacides sont obtenus par saponification des hydroxyesters précédents.

EP 0 497 678 A1

La présente invention a pour objet des diaryl-2,5 oxadiazoles-1,3,4 hydroxyester, hydroxyacide et acétoxyacide de formule :

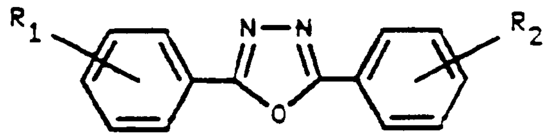

dans laquelle $R_1$ représente un radical hydroxyle ou acétyle et $R_2$ représente un radical carboxyle et où $R_1$ et $R_2$ occupent de préférence simultanément les positions 3 et 3′ ou 4 et 4′ ainsi que le dihydrazide diaromatique de formule :

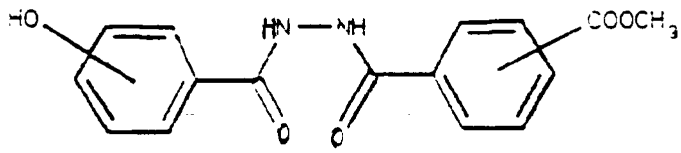

Les hydroxyesters sont préparés par cyclisation du dihydrazide diaromatique correspondant et les hydroxyacides sont obtenus par saponification des hydroxyesters précédents.

Dans la littérature, on a décrit la synthèse des diaryl-2,5 oxadiazoles-1,3,4 dihydroxy et diacides ($R_1$ = $R_2$ = OH ou COOH) et leur utilisation comme monomères de polymères thermotropes (D.BRYDON et al., Polymer, 1989, vol 30, April, pages 619 - 627).

La synthèse du bis(hydroxy-2 phényl)-2,5 oxadiazole-1,3,4 est décrite dans le brevet US 2 838 520.

Dans la demande de brevet GB 746.047, on a décrit la préparation de bis (carboxy-3 phényl)-2,5 oxadiazole-1,3,4.

Les hydroxyesters selon l'invention sont préparés par cyclisation du dihydrazide correspondant de formule :

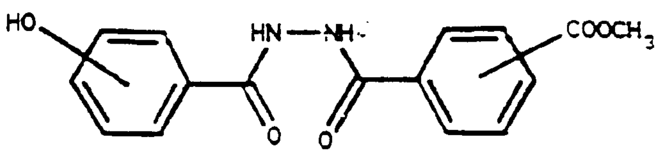

en oxadiazole à l'aide de $SOCl_2$ en présence de traces de pyridine.

Le dit dihydrazide peut être obtenu par condensation de l'hydrazide de l'acide p, o ou m-hydroxybenzoïque :

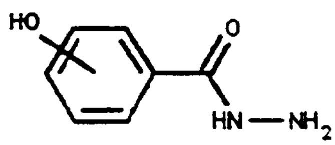

sur le chlorure d'acide de formule :

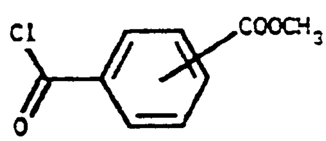

par exemple, selon le mode opératoire de D.L. BRYDON, J.S. FISHER, Polymer, vol 30 page 619 (1989).

L'hydrazide d'acide hydroxybenzoïque peut être obtenu de façon connue par réaction de l'hydrate d'hydrazine sur un ester de l'acide hydroxybenzoïque tel que l'hydroxybenzoate de méthyle.

Le chlorure d'acide peut être préparé de manière classique par réaction du chlorure de thionyle sur un

monoester d'acide benzène dicarboxylique, à température ambiante, le monoester pouvant être lui-même synthétisé par monosaponification du diester correspondant selon le mode opératoire de B.W. HOTTEN, Industrial and Engineering Chemistry, Vol. 49, n°10, page 1691 (1957).

Les hydroxyacides peuvent être préparés par saponification des hydroxyesters précédemment synthétisés. L'acétylation des hydroxyacides peut être effectuée en milieu anhydride acétique.

Les diaryl-2,5 oxadiazoles-1,3,4 hydroxyacides et leurs dérivés acétylés selon l'invention peuvent être utilisés comme monomères et notamment, ce sont des monomères particulièrement intéressants pour la synthèse de polymères thermotropes.

Les exemples suivants illustrent l'invention sans toutefois la limiter.

Dans tout ce qui suit, la signification des abréviations utilisées est la suivante :

F° = Température de fusion
Eb° = Température d'ébullition
litt = de la littérature
exp = expérimental.

**EXEMPLE 1 - SYNTHESE DE L'HYDRAZIDE DE L'ACIDE P-HYDROXYBENZOIQUE**

Mode opératoire :

Dans un réacteur de 0,5 l agité et équipé pour le reflux, on charge 1 M (152 g) de p-hydroxybenzoate de méthyle dans 100 cm³ d'éthanol. Puis on coule sous agitation 1,5 M (75 g) d'hydrate d'hydrazine 100 % à température ambiante. En fin de coulée, on porte le milieu à reflux (≈ 90°C) pendant 7 h jusqu'à disparition complète de l'ester. En fin de réaction on refroidit et on filtre le solide qui a cristallisé. Le produit est lavé à l'eau froide et séché. On obtient 123 g d'hydrazide pur à 99 % soit un rendement de 81%.

Caractéristiques du produit :

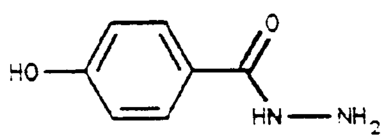

F°litt : 265°C
F°exp : 258°C - décomposition

**EXEMPLE 2 - SYNTHESE DU MONOETHYLTEREPHTALATE**

Mode opératoire :

D'après B.W. HOTTEN, Industrial and Engineering Chemistry, vol. 49, n°10, p. 1691 (1957).

Dans un réacteur agité de 2 l, on place 0,75 M (145,5 g) de diméthyltéréphtalate avec 1125 cm³ de toluène. La suspension est portée à 60°C jusqu'à dissolution totale du téréphtalate. Puis on coule une solution de potasse méthanolique (0,75 M (49,4 g) KOH 85% dans 240 cm³ de méthanol). La coulée a lieu en 20 mn puis on porte à reflux 1 heure supplémentaire. En fin de réaction, on filtre le sel formé et on le lave par des fractions de toluène chaud (50°C) pour éliminer les traces de diester. Le sel est séché et acidifié par 65 cm³ de HCl 12 N. L'ester acide obtenu est lavé à l'eau et séché. On obtient 129,5 g de produit, soit un rendement de 95 % à partir du diester.

Caractéristiques du produit :

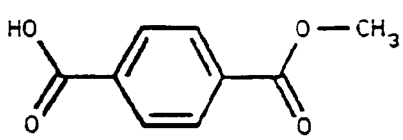

F°litt : 221°C - 223°C

F°exp : 221°C - 223°C

**EXEMPLE 3 - SYNTHESE DU CHLORURE D'ACIDE DU METHYLTEREPHTALATE**

Mode opératoire :

Dans un réacteur de 250 cm³ équipé pour le reflux, on place 0,2 M (36 g) de monométhyltéréphtalate, puis on coule 45 cm³ de $SOCl_2$ à température ambiante. Le milieu est ensuite porté à reflux jusqu'à ce que le dégagement de $SO_2$ et d'HCl cesse (soit environ 4 h). En fin de réaction, on distille l'excès de $SOCl_2$ et on récupère le chlorure d'acide (39,5 g) soit un rendement de 99 %.

Caractéristiques du produit :

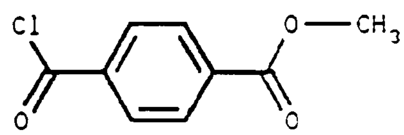

F°litt : 38°C - 40°C
F°exp : 53°C - 54°C
Eb : 135-137°C/12 mm Hg

**EXEMPLE 4 - SYNTHESE DU DIHYDRAZIDE : (hydroxy-4 benzoyl)-1 (méthoxy carbonyl-4 benzoyl)-2 hydrazine**

Mode opératoire :

D'après D.L. BRYDON, J.S. FISHER; Polymer, vol. 30, p.619 (1989).

Dans un réacteur de 2 l équipé pour le reflux, on place 108 g (0,71 M) d'hydrazide de l'acide p-hydroxybenzoïque en suspension dans 790 cm³ de dioxanne avec 37,6 g (0,355 M) de $Na_2CO_3$. Puis on coule à température ambiante 141,9 g (0,71 M) du chlorure d'acide précité en solution dans 350 cm³ de dioxanne. La coulée se fait en 45' puis on porte à reflux à 70°C pendant 9 heures jusqu'à disparition du chlorure d'acide. Le milieu est refroidi à l'ambiante. Le précipité est filtré, lavé par une solution aqueuse d'HCl 4% pour éliminer éventuellement l'hydrazide restant puis rincé 2 fois à l'eau. On obtient ainsi 184 g d'un produit de pureté > 95% (1°jet). Par concentration du filtrat, on obtient un rendement total de 87% en produit pur.(99%)

Caractéristiques du produit :

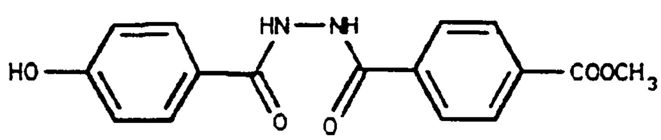

F°exp : 238°C - 240°C
RMN : conforme à la structure en RMN $H^1$ et $C^{13}$

**EXEMPLE 5 - SYNTHESE DE L'HYDROXYESTER A MOTIF OXADIAZOLE : (hydroxy-4 phényl)-2 (méthoxy carbonyl-4 phényl)-5 oxadiazole-1,3,4 :**

Mode opératoire :

Dans un réacteur de 1 l équipé pour le reflux, on place 451,5 g de $SOCl_2$ et 9 g de pyridine puis on ajoute par portion à température ambiante 89,8 g (0,286 M) de dihydrazide. L'introduction dure 20 mn. Puis on porte à reflux à 80°C pendant 5 h 55. On piège l'HCl et le $SO_2$ qui se dégagent et distille le $SOCl_2$ qui n'a pas réagi. Le produit solide est essoré et lavé à l'eau. On obtient ainsi 77 g de produit sec de pureté > 98 % en RMN, soit un rendement de 90 %.

Caractéristiques du produit :

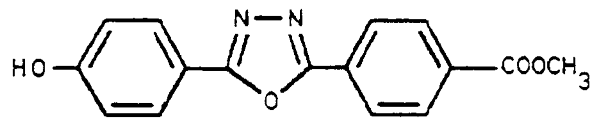

F°exp : 243°C - 244°C
RMN : conforme en RMN $H^1$ et $C^{13}$

micro analyse

| | C | H | N | O | Σ |
|---|---|---|---|---|---|
| exp | 62,67 | 4,00 | 9,25 | 22,65 | 98,57 |
| théorie | 64,79 | 4,04 | 9,44 | 21,50 | 99,86 |

**EXEMPLE 6 -SYNTHESE DE L'HYDROXYACIDE A MOTIF OXADIAZOLE -: (hydroxy-4 phényl)-2 (carboxy-4 phényl)-5 oxadiazole-1,3,4**

Mode opératoire :

Dans un réacteur équipé pour le reflux, on place 65 g (0,22 M) d'hydroxyester en suspension dans 650 g de propanol 1. On porte à reflux et on coule une solution aqueuse de potasse (31 g dans 100 g $H_2O$) en 1 h 30, puis on maintient à reflux 3 h 20.

On vérifie par potentiométrie que la saponification est terminée, puis on ajoute à chaud 43,5 $cm^3$ d'HCl 12 N. Le produit est filtré et lavé. On obtient 2 jets de 53,9 g et 5,21 g, ce qui donne un rendement de 80% en produit pur.

Caractéristiques du produit :

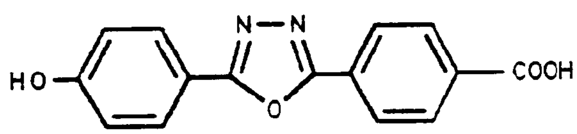

F°exp : 305°C - 308°C
RMN : conforme en RMN $H^1$ et $C^{13}$

micro-analyse

| | C | H | N | O | Σ |
|---|---|---|---|---|---|
| exp | 63,32 | 3,62 | 9,68 | 23,47 | 100,09 |
| théorie | 63,76 | 3,54 | 9,91 | 22,67 | 99,88 |

**EXEMPLE 7 - SYNTHESE DE L'ACETOXYACIDE A MOTIF OXADIAZOLE : (acétoxy-4 phényl)-2 (carboxy-4 phényl)-5 oxadiazole-1,3,4**

Mode opératoire :

Dans un réacteur équipé pour le reflux, on place 15 g (0,053 M) d'hydroxyacide dans 94 g d'anhydride acétique. On porte à reflux pendant 30 mn (110°C). On laisse refroidir et on précipite à l'eau. On obtient ainsi 15,27 g de produit sec, soit un rendement poids de 89%

Caractéristiques du produit :

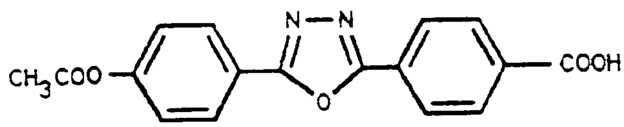

F° : 276°C - 277°C

**EXEMPLE 8 - SYNTHESE DU DIHYDRAZIDE : (hydroxy-3 benzoyl)-1 (méthoxycarbonyl-4 benzoyl)-2 hydrazine**

Ce composé est obtenu selon le mode opératoire de l'exemple 4 avec un rendement de 90 % en remplaçant l'acide p-hydroxybenzoïque par l'acide m-hydroxybenzoïque.

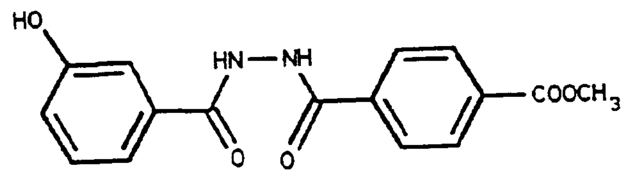

F° exp : 226 ° - 228 °C
RMN : conforme à la structure en RMN $H^1$ et $C^{13}$ .

**EXEMPLE 9 - SYNTHESE DE L'HYDROXYESTER A MOTIF OXADIAZOLE : (hydroxy-3 phényl)-2 (méthoxy carbonyl-4 phényl)-5 oxadiazole-1,3,4**

Ce composé est obtenu suivant l'exemple 5 avec un rendement de 84 % à partir du dihydrazide de l'exemple 8.

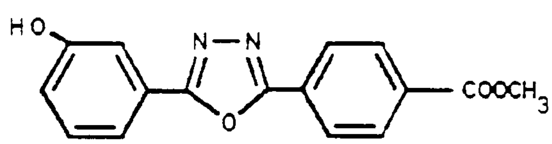

F° exp : 225° - 229°C
RMN : conforme à la structure en RMN $H^1$ et $C^{13}$

**EXEMPLE 10 - SYNTHESE DE L'HYDROXYACIDE A MOTIF OXADIAZOLE : (hydroxy-3 phényl)-2 (carboxy-4 phényl)-5 oxadiazole-1,3,4**

Ce produit est obtenu suivant l'exemple 6 avec un rendement de 81% à partir de l'hydroxyester de l'exemple 9

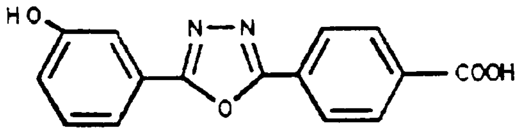

F° exp : 312° - 316°C
RMN : conforme à la structure en RMN $H^1$ et $C^{13}$

## Revendications

1. Dihydrazides diaromatiques caractérisés en ce qu'ils répondent à la formule :

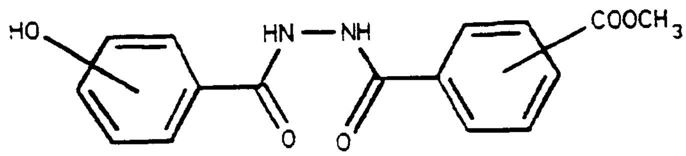

2. Diaryl-2,5 oxadiazoles-1,3,4 hydroxyesters, hydroxyacides et acétoxyacides caractérisés en ce qu'ils répondent à la formule :

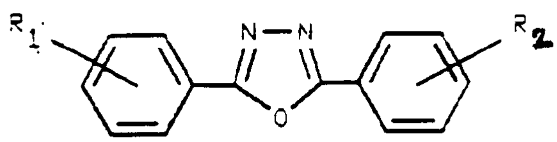

dans laquelle $R_1$ représente un radical hydroxyle ou acétyle et $R_2$ représente un radical carboxyle et où $R_1$ et $R_2$ occupent de préférence simultanément les positions 3 et 3′ ou 4 et 4′

3. Procédé de préparation des dihydrazides aromatiques de la revendication 1 par condensation de l'acide p, o ou m-hydroxybenzoïque sur le chlorure d'acide de formule :

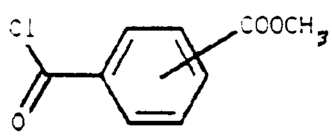

4. Procédé de préparation des diaryl oxadiazoles hydroxyesters de la revendication 2 par cyclisation des dihydrazides diaromatiques de la revendication 1 à l'aide de chlorure de thionyle et de préférence en présence de pyridine.

5. Procédé de préparation des diaryl oxadiazoles hydroxyacides de la revendication 2 par saponification des hydroxyesters de la revendication 2.

6. Procédé de préparation des diaryl oxadiazoles acétoxyacides de la revendication 2 par acétylation des hydroxyacides de la revendication 2, de préférence en milieu anhydride acétique.

7. Utilisation des hydroxyacides et des acétoxyacides de la revendication 2 comme monomères de (co)polymères, de préférence thermotropes.

Office européen des brevets

# RAPPORT DE RECHERCHE EUROPEENNE

Numero de la demande

EP 92 40 0204

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | CHEMICAL ABSTRACTS, vol. 88, no. 24, 12 Juin 1978, Columbus, Ohio, US; 'Heat stabilizers for polyolefins in contact with copper' page 44 ; * abrégé * & JP-A-52 130 841 (UBE INDUSTRIES LTD) | 1,3 | C07C243/38<br>C07D271/10 |
| X | CHEMICAL ABSTRACTS, vol. 71, no. 7, 18 Août 1969, Columbus, Ohio, US; TKACH, V. P. ET AL.: 'Synthesis of hydroxy derivatives of 2,5-diaryl-1,3,4-oxadiazoles' page 291 ; * abrégé * & KHIM. GETEROTSIKL. SOEDIN. vol. 2, page 220; | 1-6 | |
| | | | DOMAINES TECHNIQUES RECHERCHES (Int. Cl.5 ) |
| | | | C07C<br>C07D |

Le présent rapport a été établi pour toutes les revendications

| Lieu de la recherche | Date d'achèvement de la recherche | Examinateur |
|---|---|---|
| LA HAYE | 04 MAI 1992 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)